(19) Europäisches Patentamt
European Patent Office
Office européen des brevets

(11) Numéro de publication : 0 338 939 B1

# (12) FASCICULE DE BREVET EUROPEEN

(45) Date de publication du fascicule du brevet : **27.01.93 Bulletin 93/04**

(51) Int. Cl.$^5$ : **C07D 233/64,** A61K 31/415

(21) Numéro de dépôt : **89401143.6**

(22) Date de dépôt : **21.04.89**

## (54) Dérivé de l'histamine, sa préparation et son application en thérapeutique.

(30) Priorité : **22.04.88 FR 8805399**

(43) Date de publication de la demande : **25.10.89 Bulletin 89/43**

(45) Mention de la délivrance du brevet : **27.01.93 Bulletin 93/04**

(84) Etats contractants désignés : **AT BE CH DE ES FR GB GR IT LI LU NL SE**

(56) Documents cités : **EP-A- 0 214 058**

(73) Titulaire : **INSTITUT NATIONAL DE LA SANTE ET DE LA RECHERCHE MEDICALE (INSERM)**
**101, rue de Tolbiac**
**F-75654 Paris Cédex 13 (FR)**
Titulaire : **BIOPROJET**
**30, rue des Francs-Bourgeois**
**F-75003 Paris (FR)**

(72) Inventeur : **Arrang, Jean-Michel Rés. du Château Courcelles**
**Bat. 11 160 Avenue du Général Leclerc**
**F-91190 Gif Sur Yvette (FR)**
Inventeur : **Garbarg, Monique**
**10 rue Théodore de Banville**
**F-75017 Paris (FR)**
Inventeur : **Schunack, Walter**
**Königin-Luise-Strasse 2+4**
**W-1000 Berlin 33 (DE)**
Inventeur : **Schwartz, Jean-Charles**
**9 Villa Seurat**
**F-75014 Paris (FR)**
Inventeur : **Lipp, Ralph Ortwin**
**Königin-Luise-Strasse 2+4**
**W-1000 Berlin 33 (DE)**

(74) Mandataire : **Varady, Peter et al**
**Cabinet Lavoix 2, Place d'Estienne d'Orves**
**F-75441 Paris Cedex 09 (FR)**

EP 0 338 939 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet un nouveau dérivé de l'histamine, un procédé pour le préparer et son application en thérapeutique.

EP-A-0 214 058 décrit une composition pharmaceutique contenant comme principe actif un dérivé de l'histamine répondant à la formule

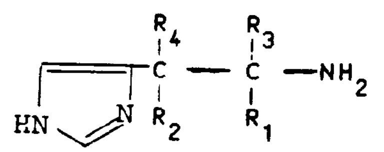

I

dans laquelle $R_1$, $R_2$ et $R_4$ désignent chacun un atome d'hydrogène ou un groupe méthyle, ou bien $R_1$ et $R_2$, pris ensemble, forment un groupe méthylène, et $R_3$ représente un atome d'hydrogène ou un groupe méthyle ou carboxy, sous réserve que, $R_1$, $R_2$, $R_3$ et $R_4$ ne désignent pas simultanément l'hydrogène, et un véhicule ou excipient pharmaceutiquement acceptable.

Ce document concerne plus particulièrement des composés déjà connus en tant que tels, mais dont on a découvert les propriétés agonistes sur les récepteurs $H_3$ régissant la libération et la synthèse de l'histamine. On a maintenant trouvé de manière inattendue que l'α,β-diméthylhistamine, composé compris dans la formule générale I mais non décrit jusqu'à présent, possède une activité agoniste sur les récepteurs $H_3$ plus sélective et supérieure à celle des autres composés de structure voisine.

L'invention a donc pour objet l'α,β-diméthylhistamine, de formule

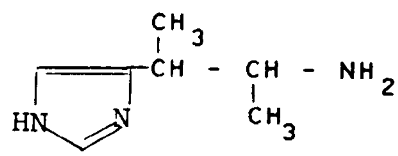

sous forme racémique, d'isomère optique ou de mélange diastéréoisomérique, ainsi que ses sels d'addition à des acides pharmaceutiquement acceptables.

Le composé ayant deux atomes de carbone asymétriques dans sa chaîne latérale, existe sous forme de quatre isomères optiques.

Un procédé de préparation du composé de l'invention est caractérisé en ce que l'on réduit un 2-amino 3-(1H-imidazol-4-yl)-butyrate d'alkyle (en $C_1$-$C_6$) à l'aide d'un hydrure métallique en 2-amino 3-(1H-imidazol-4-yl)-butanol, on transforme ce dérivé de butanol en 2-amino 1-chloro 3-(1H-imidazol-4-yl)-butane à l'aide d'un agent de chloration, et on réduit catalytiquement ce dérivé chloré.

Le 2-amino 3-(1H-imidazol-4-yl)-butyrate d'alkyle (notamment de méthyle) de départ dans ce procédé peut être préparé par exemple à partir de (1-triphénylméthyl-1H-imidazol-4-yl)carbaldéhyde selon J. L. Kelley, C.A. Miller et Ed. W. McLean, J. Med. Chem. 20, 721 (1977), et ce dernier composé est accessible à partir de (1H-imidazol-4-yl)méthanol selon P. Dziuron et W. Schunack, Arch. Pharm. (Weinheim, D.) 306, 347 (1973) et M. Barnabé et A. Burger, J. Med. Chem. 14, 883 (1971).

L'invention concerne également un procédé de préparation stéréospécifique du composé, caractérise en ce que l'on transforme un (1H-imidazol-4-yl)-carboxylate d'alkyle (en $C_1$-$C_6$), dont le reste imidazole est protégé (notamment par un groupe triphénylméthyle), en érythro-α,β-diméthylhisamine et on sépare les énantiomères (+) et (-) de ce composé au moyen d'un acide optiquement actif.

L'exemple suivant illustre la synthèse du composé selon l'invention sous forme racémique, notam-ment à partir de l'étape i).

EXEMPLE 1

a) (1H-imidazol-4-yl)méthanol.

On ajoute progressivement 52,06 g (0,5 mole) d'acétate de formamidine et 45,04 g (0,5 mole) de 1,3-dihydroxy propan-2-one à 250 ml d'ammoniac liquide. On introduit le mélange dans un autoclave en acier et le

laisse réagir pendant 12 h à 40°C, 10 bar (1MPa). Ensuite le mélange réactionnel est séparé de l'ammoniac par évaporation. On dissout le sirop jaune résultant dans 500 ml de 2-propanol et on le porte à pH2 avec du gaz HCl sec. On filtre le précipité volumineux formé et on l'extrait avec 500 ml de 2-propanol très chaud en 3 portions. L'addition de 200 ml d'éther diéthylique aux filtrats rassemblés et conservation à -25°C fournit 57,1 g (Rt : 84,9 %), de chlorhydrate de (1H-imidazol-4-yl)méthanol sous forme de cristaux brun-clair;
P.F. : 102°C.
Spectométrie de masse (SM) : m/z (int. rel. [%]) = 98 ($M^+$,47),97 (53),70 (15), 69 (44), 17 (100).
$^1$H-RMN : δ = 4.51 (s) 2H,
([$D_6$]DMSO, TMS∗)
7.47 (d) 1H,
9.02 (d) 1H, ppm.
∗ Deuterodiméthylsulfoxyde, tétraméthylsilane.

b) (1-triphénylméthyl-1H-imidazol-4-yl)méthanol

A une solution agitée de 18,8 g (140 mmole) de (1H-imidazol-yl)méthanol dans 140 ml de diméthylformamide et 50 ml de triéthylamine, on ajoute goutte à goutte 40,5 g (145 mmole) de chlorure de triphénylméthyle dans 300 ml de diméthylformamide. Après 2 h d'agitation a température ambiante, on verse le mélange réactionnel sur 2 kg de glace pilée. On lave le précipité avec de l'eau et on enlève les impuretés par digestion avec 400 ml de dioxanne chaud. Aprés récupération, lavage avec de l'éther diéthylique et séchage sous vide on obtient 41 g (Rt : 86%) de (1-triphénylméthyl-1H-imidazol-4-yl)-méthanol sous forme d'une poudre blanche;
P.F. : 224 - 227°C
SM : m/z (int. rel. [%]) = 340 ($M^+$, <1), 243 (100), 165 (64).
$^1$H-RMN : δ = 3.14 (br) 1H, remplaçable par $D_2O$,
([$D_6$]DMSO,TMS)
4.56 (s) 2H,
6.77 (d) 1H,
7.20 - 7.40 (m) 15H,
7.55 (d) 1H, ppm.

c) (1-triphénylméthyl-1H-imidazol-4-yl)carbaldéhyde

On chauffe au reflux, 68,1 g (0,2 mol) de (1-triphénylméthyl-1H-imidazol-4-yl)-méthanol et 177,9 g (2 mole) de dioxyde de manganèse pendant 3 h dans 1,3 l de dioxanne. On filtre la suspension chaude sur 100 ml de Célite sur un Buchner et la lave avec 0,5 l de dioxanne chaud en trois portions. On évapore la solution sous vide et on sèche le résidu solide blanc sous vide. On obtient 49,1 g (72,5%) de produit;
P.F. : 188 - 193°C.
SM : m/z (int. rel. [%]) = 338 ($M^+$, <1), 243 (100), 165 (56).
$^1$H-RMN δ = 7.01 - 7.43 (m) 15H,
([$D_6$]DMSO,TMS)
7.53 (d) 1H,
7.61 (d) 1H,
9.87 (s) 1H, ppm.

d) 1-(1-triphénylméthyl-1H-imidazol-4-yl) éthanol

A une solution fraichement préparée de 290 mmole de iodure de méthyl-magnésium dans 700 ml d'éther diéthylique on ajoute 49,1 g (145 mmole) de (1-triphénylméthyl-1H-imidazol-4-yl)-carbaldéhyde dans 200 ml de tetrahydrofuranne. On laisse le mélange réagir pendant 2 h à température ambiante, puis on ajoute une solution de 19,4 g (0,36 mole) de chlorure d'ammonium dans 70 ml d'eau. Après agitation une nuit, on sépare le précipité par filtration et on lave avec 300 ml de tétrahydrofuranne. Les filtrats combinés sont dilués avec 300 ml d'éther diéthylique et lavés avec de l'eau. On sèche la phase organique sur sulfate de sodium. Après évaporation, on obtient 46,5 g (Rt : 90,4%) de 1-(1-triphénylméthyl-1H-imidazol-4-yl)-éthanol sous forme d'une poudre blanche. P.F. : 156 - 157°C.
SM : m/z (int. rel. [%]) = 354 ($M^+$, <1), 243 (100), 165 (50).

e) 1-chloro 1-(1H-imidazol-4-yl)éthane

On dissout 46,1 g (130 mmole) de 1-(1-triphénylméthyl-1H-imidazol-4-yl)éthanol dans 500 ml d'acide chlorhydrique 2N à 70°C pendant 1 h. On sépare le précipité par filtration. On lave la solution avec 300 ml d'éther diéthylique en deux portions et on évapore sous vide pour obtenir le chlorhydrate de 1-(1H-imidazol-4-yl)éthanol sous forme d'une huile légèrement jaune. On ajoute goutte à goutte 100 ml de chlorure de thionyle à cette huile à 0°C et laisse réagir la solution à température ambiante une nuit. L'évaporation et la cristallisation dans l'éther diéthylique/éther diméthylique de l'éthylèneglycol fournit 19,1 g (88%) de chlorhydrate de 1-chloro 1-(1H-imidazol-4-yl)éthane. Cristaux jaunes, P.F. : 101-104°C.

SM : m/z (int. rel. [%] = 130 ($M^+$,<1), 95 (72), 94 (74), 38 (100).

$^1$H-RMN δ = 1.88 (d) 3H,
([$D_6$] DMSO, TMS)
5.51 (q) 1H,
7.77 (d) 1H,
9.19 (d) 1H, ppm.

f) 2-acétamido 2-[1-(1H-imidazol-4-yl)éthyl] propane-1,3-dicarboxylate de diéthyle

On ajoute 11,9 g (55 mmole) d'acétamidomalonate de diéthyle à une solution de 105 mmole d'éthylate de sodium à 100 ml d'éthanol. Après 2h d'agitation on refroidit la solution à 0°C et on ajoute 8,4 g (50 mmole) de chlorhydrate de 1-chloro 1-(1H-imidazol-4-yl)-éthane. Le mélange est agité pendant 2 h, concentré sous vide et dissous dans 100 ml d'acide chlorhydrique à 2%. On extrait cette solution avec 50 ml d'éther diéthylique, on porte à pH8 par addition de carbonate de sodium et on extrait avec 200 ml de chlorure de méthylène en quatre portions. On rassemble les couches de chlorure de méthylène, sèche sur sulfate de sodium et évapore sous vide, pour obtenir 13,8 g (88,7%) de 2-acétamido-2-[(1-H-imidazol-yl)éthyl]propane-1,3-dicarboxylate de diéthyle sous forme d'une huile brun-clair.

SM : m/z (int. rel. [%]) = 311 ($M^+$, 48), 252 (60), 238 (43), 196 (54), 174 (42), 160 (56), 148 (61), 96 (79), 95 (90), 29 (100).

$^1$H-RMN δ = 0.99 - 1.44 (m) 9H,
([$D_6$]DMSO, TMS)
1.95 (s) 3H,
3.62 - 4.14 (m) 5H,
6.84 - 6.96 (m) 1H,
7.69 (d) 1H,
7.98 (br) 1H, remplaçable par $D_20$, ppm.

g) Acide 2-amino 3-(1H-imidazol-4-yl)butyrique

On chauffe 13,1 g (42 mmole) de 2-acétamido-2-[1-(1H-imidazol-4-yl)éthyl]propane-1,3-dicarboxylate de diéthyle sous reflux pendant 12 h dans 100 ml d'acide chlorhydrique concentré. Après refroidissement et évaporation sous vide, on obtient 9,8 g (96%) de dichlorhydrate de l'acide 2-amino 3-(1H-imidazol-4-yl)butyrique sous forme de mousse.

$^1$H-RMN δ= 1.32 - 1.42 (m) 3H,
([$D_6$]DMSO, TMS)
3.61 - 3.69 (m) 1H,
4.36 - 4.46 (m) 1H,
7.54 (d) 1H,
8.5 - 9.1 (br) 3H, remplaçable par $D_2O$,
9.16 (d) 1H,
14.75 (br) 2H, remplaçable par $D_2O$, ppm.

h) 2-amino 3-(1H-imidazol-4-yl)butyrate de méthyle

On dissout 9,40 g (38,9 mmole) de dichlorhydrate d'acide 2-amino 3-(1H-imidazol-4-yl)butyrique dans 100 ml de méthanol. On fait barboter du gaz HCl à travers la solution chauffée au reflux pendant 4 h. Aprés refroidissement, l'acide chlorhydrique en excès est chassé sous vide. On obtient 9,8 g (98%) de dichlorhydrate de 2-amino 3-(1H-imidazol-4-yl)butyrate de méthyle sous forme d'un solide amorphe.

SM : m/z (int. rel. [%]) = 183 ($M^+$, <1), 124 (20), 109 (6), 96 (94), 95 (100).

$^1$H-RMN (base) : δ = 1.09 - 1.27 (m) 3H, ([$D_6$]DMSO, TMS)
2.89 - 3.72 (m) 5H,
5.84 (br) 2H, remplaçable par $D_2O$,
6.76 (d) 1H,
7.49 (d) 1H, ppm.

i) 2-amino 3-(1H-imidazol-4-yl)-butanol

On ajoute 7,7 g (30 mmole) de dichlorhydrate de 2-amino 3-(1H-imidazol-4-yl)butyrate de méthyle à 0°C, à une suspension agitée de 3,4 g (90 mmole) d'hydrure de lithium aluminium dans 125 ml de tétrahydrofuranne. Ensuite on chauffe le mélange sous reflux pendant 3h, refroidit à 0°C et on hydrolyse par addition de 6,5 ml d'eau dans 15 ml de tétrahydrofuranne. On agite avec 20 ml d'une solution d'hydro-xyde de sodium 5N pour obtenir un précipité à gros grains que l'on filtre et extrait par 100 ml d'éthanol en trois portions. La concentration des fractions fournit une huile que l'on traite avec de l'éthanol anhydre pour séparer la matière minérale. Après filtration et évaporation on obtient 4g (Rt : 85,9%) de 2-amino 3-(1H-imidazol-4-yl)butanol qui est transformé en son dichlorhydrate huileux. On prélève un échantillon pour analyse, on le transforme en dipicrate que l'on recristallise dans un mélange éthanol/eau. Cristaux jaunes, P.F. : 168°C.
$C_7H_{13}N_3O.2\ C_6H_3N_3O_7$ (613,4) masse molaire : 155 (SM)
Calc.: C 37,20 H 3,12 N 20,55
Trouvé: C 37,23 H 3,08 N 20,40
$^1$H-RMN : δ= 1.32 (m) 3H,
([$D_6$])DMSO, TMS)
3.26 - 3.79 (m) 5H, 1H remplaçable par $D_2O$,
7.49 - 7.56 (m) 1H,
7.87 (br) 3H, remplaçable par $D_2O$,
8,63 (s) 4H,
9,12 (d) 1H, ppm.

j) 2-amino 1-chloro-3-(1H-imidazol-4-yl)butane

On dissout 3 g (13,2 mmole) 2-amino-3-(1H-imidazol-4-yl)butanol, dichlorhydrate dans un mélange de 25 ml de tétraméthylènesulfone et 10 ml de chlorure de thionyle et on agite 12 h à température ambiante. L'addition goutte à goutte de 200 ml de chloroforme fournit du dichlorhydrate de 2-amino 1-chloro-3-(1H-imidazol-4-yl)butane. On obtient 2,87 g (Rt : 88,2%) d'un précipité hygroscopique.
$C_7H_{12}ClN_3.2HCl$ (246,6)
SM : m/z (int. rel. [%]) = 173 ($M^+$,<1), 137 (7), 96 (98), 95 (100).
$^1$H-RMN : δ = 1.37 (d) 3H,
([$D_6$]DMSO, TMS)
3.42 - 3.55 (m) 1H,
3.77 - 3.86 (m) 1H,
4.03 (d) 2H,
7.49 - 7.56 (m) 1H,
8.75 (br) 3H, remplaçable par $D_2O$,
9,15 (d) 1H,
14.7 (br) 2H, remplaçable par $D_2O$ ppm.
Un échantillon pour analyse est transformé en dipicrate, cristaux jaunes, P.F. : 196 -198°C (éthanol/eau).
$C_7H_{12}ClN_3.2\ C_6H_3N_3O_7$ (631,9)
Calc. : C 36,12 H 2,87 N 19,95
Trouvé : C 36,25 H 2,76 N 19,86

k) [3-(1H-imidazol-4-yl)but-2-yl]amine (RR, SS,RS,SR) ou α,β-diméthylhistamine

On hydrogène un mélange de 2 g (8,1 mmole) de dichlorhydrate de 2-amino 1-chloro-3-(1H-imidazol-4-yl)butane, 1,3 g (16,2 mmole) d'acétate de sodium et 100 ml d'acide acétique à 10% sur 0,5 g de palladium à 10% sur charbon activé pendant 10 jours sous 10 bars (1MPa), à température ambiante. On sépare le catalyseur par filtration et porte le filtrat de pH1 par addition d'acide chlorhydrique concentré. Après évaporation, on dissout le résidu huileux.dans de l'éthanol sec et on sépare la matière minérale par filtration. Par addition d'éther de pétrole, le composé du titre cristallise sous forme de dichlorhydrate. Une recristallisation du méthanol/acétonitrile fournit 0,72 g (Rt : 40,4%) de [3-(1H-imidazol-4-yl)but-2-yl] amine. 2HCl.O,25 $CH_3OH$ sous forme de cristaux incolores.
P.F. : 249 - 254°C (décomp.).
$C_7H_{13}N_3$.2HCl.O,25 $CH_4O$ (220,1)
Calc. : C 39,56 H 7,33 N 19,09
Trouvé : C 39,50 H 7,40 N 19,15
SM : m/z (int.rel. [%]) = 140 (11), 139 ($M^+$, 1), 124 (14), 96 (95), 81 (35), 44 (100).
$^1$H-RMN δ = 1.10 - 1.36 (m) 6H,
([$D_6$]DMSO, TMS)
3.28 (m) 1H,
3.40 (m) 1H,
7.51 - 7.58 (m) 1H,
8.36 (br) 3H, remplaçable par $D_2O$,
9,12 (d) 1H,
14,76 (br) 2H, emplaçable par $D_2O$ ppm.

L'exemple suivant illustre la synthèse stéréospécifique du composé selon l'invention.

EXEMPLE 2

a) (1H-imidazol-4-yl)-carboxylate de méthyle

On ajoute 112,09g (1 mole) d'acide (1H-imidazol-4-yl)carboxylique à 1,3 l de méthanol. On fait barboter du HCl gazeux à travers la solution chauffée au reflux pendant 4h. Après refroidissement à température ambiante et évaporation jusqu'à 300 ml on obtient 98g de chlorhydrate du composé du titre sous forme de cristaux blancs. Une nouvelle évaporation fournit 46,5 g de produit supplémentaire (au total : 144,5 g, Rdt 88,9%). On recristallise un échantillon pour analyse dans le méthanol, P.F. : 173-174°C.
$C_5H_6N_2O_2$.HCl (162,6)
Calc.: C 36,94 H4,34 N 17,23
C 36,93 H 4,44 N 17,23
SM : m/z (int. rel. [%] = 126 ($M^+$, 63), 95 (100), 67 (41), 40 (68).
$^1$H-RMN : δ= 12,97 (br) 2H, remplaçable par $D_2O$,
(/D6/DMSO,TMS)
9,29 (d) 1H,
8,37 (d) 1H,
3,90 (s) 3H, ppm.

b) (1-triphénylméthyl-1H-imidazol-4-yl)carboxylate de méthyle

A une solution de 151,79g (1,5 mole) de triéthylamine dans 1 l de chlorure de méthylène on ajoute 97,55g (0,6 mole) de chlorhydrate de (1H-imidazol-4-yl)carboxylate de méthyle. Après avoir agité pendant 1h à température ambiante, on ajoute au mélange goutte à goutte une solution de 183,99g (0,66 mole) de chlorure de triphénylméthyle dans 500 ml de chlorure de méthylène et on laisse réagir pendant 1h. Lorsque la réaction est terminée, on lave le mélange 4 fois avec 200 ml d'eau, sèche sur sulfate de sodium et évapore à sec. L'huile obtenue cristallise après une nuit. Après pulvérisation et 3 lavages avec 200ml d'éther diéthylique chaud on obtient 210,2g (95,1%) du composé du titre sous forme de cristaux jaune-clair. On recristallise un échantillon pour analyse; cristaux blancs, P.F. : 147-152°C.
$C_{24}H_{20}N_2O_2$ (368,5)
Calc.: C 78,24 H 5,47 N 7,60
Trouvé: C 77,96 H 5,23 N 7,66
SM : m/z (int. rel. [%]) = 368 ($M^+$, 30), 258 (100).
$^1$H-RMN : δ= 7,58 (d) 1H,
($CDCl_3$, TMS)
7,47 - 7,01 (m) 16H, 3,85 (s) 3H, ppm.

c) 3-oxo-3-(1-triphénylméthyl-1H-imidazol-4-yl)propionate d'éthyle.

On dissout 202,65g (0,55 mole) de (1-triphénylméthyl-1H-imidazol-4-yl)carboxylate de méthyle dans 0,8 l de toluène sec à 85°C. On ajoute 44 g (1,1 mole) d'hydrure de sodium (en dispersion à 60% dans de l'huile

minérale). On ajoute au mélange sous agitation 96,92g (1,1 mole) d'acétate d'éthyle goutte à goutte en 2 heures. On laisse réagir le mélange pendant 16 h à 70°C. Ensuite on chasse le toluène sous pression réduite et on utilise le mélange obtenu dans l'étape suivante sans purification. On prélève toutefois une petite quantité du résidu huileux brun pour analyse, on la dissout dans du chlorure de méthylène, lave avec une solution aqueuse de chlorure d'ammonium et de l'eau, on sèche sur sulfate de sodium et on évapore pour obtenir une huile. Une critallisation dans l'éther diéthylique et recristallisation dans l'éthanol/éther diéthylique fournit le composé du titre sous forme de cristaux blancs, P.F. : 136-139°C.

$C_{27}H_{24}N_2O_3$ (424,5)
Calc. : C 76,39 H 5,70 N 6,60
Trouvé : C 76,30 H 5,66 N 6,47
SM : m/z (int. rel. [%]) = 424 ($M^+$, <1), 224 (19), 243 (100).
$^1$H-RMN : δ= 7,62 (d) 1H,
($CDCl_3$, TMS)
7,55-6,99 (m) 16H,
4,61 (q) 2H,
3,99 (s) 2H,
1,24 (t) 3H, ppm.

d) 1-(1-triphénylméthyl-1H-imidazol-4-yl)éthanone

On dissout le résidu de l'étape c) dans un mélange de 75 g d'hydroxyde de potassium, 140 ml d'eau et 1,3 l d'éthanol. On chauffe cette solution sous reflux pendant 10h. On sépare la matière solide par filtration. Après évaporation et dissolution dans du chlorure de méthylène, on lave 3 fois avec 400 ml d'eau (l'addition d'isopropanol sépare les couches), on sèche sur sulfate de sodium et évapore. Après agitation et addition d'éther diéthylique on obtient 115,3 g du composé du titre (59,5% par rapport à b)) sous forme de cristaux jaune-clair. On recristallise un échantillon pour analyse dans l'éthanol : cristaux blancs, P.F. : 164-165°C.

$C_{24}H_{20}N_2O$ (352,4)
Calc.: C 81,79 H 5,72 N 7,95
Trouvé: C 81,67 H 5,64 N 8,03
SM : m/z (int. rel. [%]) = 352 ($M^+$, <1), 244 (22), 243 (100), 183 (85).
$^1$H-RMN : δ = 7,45 - 7,20 (m) 17H,
($CDCl_3$, TMS)
3,06 (s) 3H, ppm.

e) (Z)-2-méthyl-3-(1-triphénylméthyl-1H-imidazol-4-yl)2-buténoate d'éthyle.

On met en suspension 11,7 g (0,3 mole) d'amidure de sodium dans 300 ml de tétrahydrofuranne sous azote. On ajoute goutte à goutte 71,47 g (0,3 mole) de 2-phosphonopropionate de triéthyle au mélange sous agitation, qui est maintenu à température ambiante encore 2h. On ajoute 30,5 g (86,5 mmole) de 1-(1-triphénylméthyl-1H-imidazol-4-yl)éthanone et on porte au reflux pendant 16h. Après évaporation et dissolution dans 1 l de chloroforme/isopropanol (3:1), on lave 3 fois avec de l'eau. Après séchage sur sulfate de sodium et évaporation on obtient une huile consistant principalement en E-2-méthyl-3-(1-triphénylméthyl-1H-imidazol-4-yl) 2-buténoate d'éthyle. En traitant ce résidu avec l'éther diéthylique on obtient 10,9 g d'une matière solide consistant principalement en l'isomère Z. Une purification sur colonne de chromatographie (gel de silice 63-200 μm, éluant : éther de pétrole/éther diéthylique (2:3) fournit 5,6 g (14,8%) du composé du titre sous forme d'un solide blanc. On recristallise un échantillon pour analyse dans un mélange éther diéthylique/éthanol et obtient des cristaux incolores, P.F. : 166-168°C.

$C_{29}H_{28}N_2O_2$ (436,6)
Calc.: C 79,79 H 6,46 N 6,43
Trouvé: C 79,49 H 6,43 N 6,34
SM : m/z (int. rel. [%]) = 436 ($M^+$, 2), 244 (20), 243 (100), 165 (23).
$^1$H-RMN : δ = 7,58-7,11 (m) 16H,
($CDCl_3$, TMS)
6,76 (d) 1H,
4,07 (q) 2H,
1,98 (s) 6H,
1,17 (t) 3H, ppm.

f) érythro-3-(1H-imidazol-4-yl)2-méthylbutanoate d'éthyle

On dissout 10,2 g (23,4 mmole) de Z -2-méthyl-3-(1-triphénylméthyl-1H-imidazol-4-yl)2-buténoate d'éthyle dans 350 ml de tétrahydrofuranne et on ajoute 1,5 g de palladium à 10% sur charbon activé. On hydrogène le mélange pendant 3 jours à température ambiante et sous 10 bars (1MPa). Après séparation du catalyseur par filtration, on évapore la solution à sec. On purifie le résidu sur colonne de chromatographie (gel de silice 63-200 μm, éluant 1 : éther diéthylique, éluant 2 : chloroforme/méthanol saturé d'ammoniac (1:1), pour obtenir 3,9 g (84,9%) du composé du titre sous la forme d'une huile incolore. On transforme une petite quantité de ce composé en hydrogénomaléate : cristaux blancs, P.F- : 91-94°C (éthanol/éther diéthylique).

$C_{10}H_{16}N_2O_2.C_4H_4O_4$ (312,3)
Calc.: C 53,84 H 6,45 N 8,97
Trouvé: C 53,72 H 6,60 N 8,82
MS : m/z (int. rel. [%] = 196 ($M^+$, 11), 123 (30), 95 (100); obtenu avec un échantillon de l'HCl.
$^1$H-RMN : δ = 8,88 (s) 1H,
([D6]DMSO, TMS)
7,46 (s) 1H,
6,05 (s) 2H,
4,08 (q) 2H,
3,10 (dq) 1H,
2,70 (dq) 1H,
1,22-1,13 (m) 6H,
0,95 (d) 3H, ppm.

g) acide erythro-3-(1H-imidazol-4-yl)2-méthylbutanoïque

On dissout 3,8 g (19,4 mmole) d'érythro-3-(1H-imidazol-4-yl)2-méthylbutanoate d'éthyle dans 60 ml d'acide chlorhydrique 6N et on chauffe au reflux pendant 5h. Après évaporation on obtient le chlorhydrate de l'acide érythro-3-(1H-imidazol-4-yl)2-méthylbutanoïque sous forme d'une huile hygroscopique qui est-utilisée sans purification dans l'étape suivante.

$C_8H_{12}N_2O_2.HCl$ (204,7)
SM : m/z (int. rel. [%]) = 168 ($M^+$, 17), 123 (19), 95 (100), 68 (10)
$^1$H-RMN : δ = 9,04 (s) 1H,
([D6]DMSO, TMS)
7,51 (s) 1H,
3,10 (m) 1H,
2,63 (m) 1H,
1,24 (d) 3H,
0,93 (d) 3H, ppm.

h) érythro-[3-(1H-imidazol-4-yl)but-2-yl]amine (ou érythro-α,β-diméthylhistamine)

On ajoute 125 ml de chloroforme à une solution de chlorhydrate de l'acide érythro-3-(1H-imidazol-4-yl)2-méthylbutanoïque obtenu ci-dessus dans 20 ml d'acide sulfurique concentré. On agite le mélange et on ajoute 5,85g (90 mmole) d'azoture de sodium pendant une heure à 0°C. Ensuite on maintient le mélange réactionnel à 45°C pendant 14 h. Après addition de glace, on sépare la couche organique. On porte la phase aqueuse à pH 8,5 et évapore à sec. On traite la matière solide obtenue dans un Soxhlet avec de l'isobutanol pour obtenir le composé du titre sous forme d'une huile brute que l'on purifie en colonne de chromatographie (gel de silice 63-200 μm, éluant : chloroforme/méthanol saturé avec de l'ammoniac (85:15)), pour obtenir 2,32g (85,9% par rapport à f)) d'érythro-[3 -(1H-imidazol-4-yl)but-2-yl] amine sous forme d'une huile incolore. On convertit un échantillon pour analyse en dipicrate : cristaux jaunes, P.F. : 228-232°C (éthanol).

$C_7H_{13}N_3.2\ C_6H_3N_3O_7$ (597,4)
Calc.: C 38,20 H 3,21 N 21,10
Trouvé: C 38,22 H 3,15 N 21,14
$^1$H-RMN : δ = 9,09 (s) 1H,
([D6]DMSO, TMS)
8,60 (s) 4H,
7,81 (br) 3H, remplaçable par $D_2O$,
7,55 (s) 1H,

3,40 (m) 1H,
3,15 (m) 1H,
1,30 (d) 3H,
1,09 (d) 3H, ppm.

i) di[(+)-2S,3S-di-O-(4-toluoyl)hydrogénotartrate] de (-)-érythro-[3-(1H-imidazol-4-yl)but-2-yl]-amine, monohydrate

On dissout 0,56 g (4 mmole) d'érythro-(3-(1H-imidazol-4-yl)but-2-yl]amine dans 30ml d'une solution chaude d'éthanol/eau (1:1) et on l'ajoute à une solution chaude de 3,13g (8,1 mmole) d'acide (+)-2S, 3S-di-O-(4-toluoyl)tartrique dans 80 ml d'éthanol/eau (1:1). On abandonne 3 jours à la température ambiante, puis on recristallise les cristaux blancs résultants dans l'éthanol/eau (1:1) jusqu'à l'obtention d'un point de fusion et d'un pouvoir rotatoire constants : on obtient 0,79g (42,5%) du composé du titre sous forme de cristaux blancs, P.F. : 181°C.
$C_7H_{13}N_3.2C_{20}H_{18}O_8.H_2O$ (929,9)
Calc. : C 60,71 H 5,53 N 4,52
Trouvé : C 60,39 H 5,41 N 4,76
$[\alpha]_D^{20}$ = + 110,6(2)° (c = 0,1, $CH_3OH$).

j) (-)-érythro-[3-(1H-imidazol-4-yl)but-2-yl]amine

On dissout 0,77g (0,83 mmole)de di/(+)-2S,3S-di-O-(4-toluoyl)hydrogénotartrate) de (-)érythro-[3-(1H-imidazol-4-yl)but-2-yl]amine monohydrate dans 60 ml d'éthanol/eau (2:1) et on ajoute 0,5ml d'acide bromhydrique à 47%. Après évaporation et redissolution dans 20ml d'eau et 20ml de chlorure de méthylène, on extrait 4 fois avec du chlorure de méthylène. On évapore la solution aqueuse et sépare l'acide bromhydrique en excès en dissolvant dans l'éthanol puis évaporant plusieurs fois. Après addition d'éther diéthylique et d'isopropanol puis agitation on obtient 0,18g (72%) de dibromhydrate de (-)-érythro-[3-(1H-imidazol-4-yl)but-2-yl] amine sous forme de cristaux blancs fondant dans la plage de 207-230°C.
$C_7H_{13}N_3$.2 HBr (301,0)
Calc.: C 27,93 H 5,02 N 13,96
Trouvé: C 27,80 H 5,05 N 13,65
$^1$H-RMN : $\delta$ = 14,22 (br) 2H, remplaçable par $D_2O$,
([D6]DMSO, TMS)
9,14 (s) 1H,
7,98 (br) 3H, remplaçable par $D_2O$,
7,59 (s) 1H,
3,37 (m) 1H,
3,23 (m) 1H,
1,32 (d) 3H,
1,12 (d) 3H, ppm.

k) di[(-)-2R,3R,di-O-(4-toluoyl)hydrogénotartrate] de (+)-érythro-[3-(1H-imidazol-4-yl)but-2-yl]-amine, monohydrate

On évapore à sec le filtrat de la première cristallisation de l'étape i) et on le transforme en dibromhydrate (comme dans l'étape i)). On dissout dans l'éthanol sec et ajoute une quantité équivalente de tert-butylate de potassium pour obtenir, après filtration et évaporation, 0,17g (1,2 mmole) d'érythro-[3-(1H-imidazol-4-yl)but-2-yl]amine sous forme de base libre. On dissout la base dans 10 ml d'une solution chaude éthanol/eau (1:1) et on ajoute à une solution chaude de 1,07g (2,5 mmole) d'acide (-)-2R,3R-di-O-(4-toluyol-)tartrique dans 30 ml d'éthanol/eau (1:1). On abandonne 3 jours à la température ambiante et on recristallise les cristaux blancs résultants dans l'éthanol/eau (1:1) jusqu'à l'obtention d'un point de fusion et d'un pouvoir rotatoire constants. On obtient 0,21g (11,3 % par rapport à l'éduit de l'étape i) de cristaux blancs, P.F- :181°C.
$C_7H_{13}N_3.2\ C_{20}H_{18}O_8.H_2O$ (929,9)
Calc.: C 60,71 H 5,53 N 4,52
Trouvé: C 60,52 H 5,34 N 4,69
$[\alpha]_D^{20}$ = -109,4 (2)° (c = 0,1, $CH_3OH$)

1) (+)-érythro-[3-(1H-imidazol-4-yl)but-2-yl]amine

On transforme 0,2g (0,22 mmole) du di[(-)-2R,3R-di-O-(4-toluoyl)hydrogénotartrate]de (+)-érythro-[3-(1H-imidazol-4-yl)but-2-yl]amine, monohydrate en dibromhydrate de la manière décrite sous j) pour obtenir 60mg (86,7%) de (+)-érythro-[3-(1H-imidazol-4-yl)but-2-yl]amine sous forme de cristaux blancs fondant dans la plage de 209-230°C (éther-diéthylique/isopropanol).

$C_7H_{13}N_3$.2 HBr (301,0)
Calc. : C 27,93 H 5,02 N 13,96
Trouvé : C 27,65 H 5,07 N 13,60

Le composé de l'invention a fait l'objet d'une étude pharmacologique.

Le composé a été étudié sous forme racémique de stéréoisomère. Les tests effectués sont les suivants :

a) inhibition de la libération d'histamine induite par dépolarisation de coupes de cortex cérébral de rat selon la technique décrite par Arrang et al. (Nature, 1983, 302, 832). L'inhibition maximale induite par le composé de l'invention est identique à celle qu'induit l'histamine (c'est donc un agoniste complet) et sa concentration inhibitrice 50% ($CI_{50}$) est la suivante :

- "racémate" : $12 \times 10^{-9}$M
- forme érythro : $6 \times 10^{-9}$M
- isomère(+)-érythro : $3,4 \pm 2,0 \times 10^{-9}$M
- isomère(-)-érythro : $48 \pm 31 \times 10^{-9}$M
- forme thréo : $200 \times 10^{-9}$M

Il apparait donc que l'activité du composé de l'invention sur les récepteurs $H_3$ réside sélectivement dans l'un des quatre stéréoisomères, à savoir l'énantiomère(+)-érythro. L'activité de l'énantiomère (-)-érythro est vraisemblablement inférieure à la valeur trouvée ci-dessus, ce composé étant contaminé par une faible proportion (env. 15%) d'énantiomère(+)-érythro.

b) Contraction de l'iléon isolé de cobaye : le composé est un agoniste complet mais présente moins de 1 p. mille de l'activité de l'histamine.

c) Contraction de l'oreillette isolée de cobaye : le composé est un agoniste partiel (effet maximum : 60% de celui de l'histamine) environ mille fois moins actif que l'histamine.

Les résultats sont rassemblés dans le tableau suivant.

TABLEAU : Activité du composé de l'invention comparée à celle de l'histamine sur les trois classes de récepteurs histaminergiques.

| | $H_3$ a) | $H_1$ b) | $H_2$ c) |
|---|---|---|---|
| histamine | 100 | 100 | 100 |
| α,β-diméthylhistamine | | | |
| - "racémique" .... | 516 | 0,06 | 0,1 |
| - érythro ...... | 1030 | | |
| - (+)érythro ... | 1800 | | |
| - (-)érythro ... | 129 | | |

En outre, le composé s'est révélé actif in vivo puisqu'à la dose de 20 mg/kg (racémate) par voie orale il inhibe de manière maximale la synthèse d'histamine dans le cerveau de rat (mesurée selon la technique décrite par Arrang et al., Nature 1987, 327, 117).

Cette étude montre que le composé selon l'invention présente une activité $H_3$ très importante et surtout très sélective. En effet, son activité sur les récepteurs $H_1$ et $H_2$ est négligeable, de dix à cent fois plus faible que celle des meilleurs parmi les composés connus selon EP-A-O 214 058, tandis que son activité $H_3$ et sa

sélectivité sont supérieures. Il peut donc constituer un agoniste $H_3$ très puissant et très sélectif utilisable en thérapeutique dans des conditions de sécurité accrues.

A ce titre il est susceptible de diminuer les transmissions histaminergiques dans le tube digestif, les systèmes nerveux, cardiovasculaire et immunitaire. Il est donc utilisable en thérapeutique comme médicament à effets sédatif, régulateur du sommeil, anticonvulsivant, régulateur des sécrétions hypothalamo-hypophysaires, antidépresseur, modulateur de la circulation cérébrale, etc ...

Par ailleurs une inhibition de la libération des messagers de l'inflammation dans diverses affections allergiques (ex. : asthme) est attendue de la stimulation des récepteurs $H_3$ du poumon, par exemple.

En gastro entérologie, l'inhibition de libération d'histamine gastrique est susceptible d'exercer des effets anti-secrétoire et anti-ulcéreux. Une modification de libération des messagers des réponses immunitaires est susceptible de moduler ces dernières.

En raison de ces propriétés, le composé de l'invention, sous forme racémique ou de son stéréoisomère le plus actif peut constituer le principe actif de compositions pharmaceutiques comportant également un véhicule ou excipient pharmaceutiquement compatible. Son mode d'action, ses divers effets pharmacologiques et sa faible toxicité chez l'animal laissent prévoir des applications en médecine humaine et vétérinaire à des doses de l'ordre de 0,1 à 10 mg/kg, notamment par voie orale et parentérale.

Il peut être représenté notamment sous forme de comprimés, dragées, gélules, aérosols, solutés injectables, ou suppositoires.

## Revendications

### Revendications pour les Etats contractants suivants: AT, BE, CH, DE, FR, GB, IT, LI, LU, NL, SE

1. L'α,β-diméthylhistamine, sous forme racémique, d'isomère optique ou de mélange diastéréoisomérique, ainsi que ses sels d'addition à des acides pharmaceutiquement acceptables.

2. L'érythro-α,β-diméthylhistamine, ainsi que ses sels d'addition à des acides pharmaceutiquement acceptables.

3. L'isomère(+)-érythro-α,β-diméthylhistamine, ainsi que ses sels d'addition à ces acides pharmaceutiquement acceptables.

4. Composition pharmaceutique contenant un composé selon l'une des revendications 1 à 3 et un véhicule ou excipient pharmaceutiquement compatible.

5. Procédé de préparation du composé selon la revendication 1, caractérisé en ce que l'on réduit un 2-amino 3-(1H-imidazol-4-yl)-butyrate d'alkyle (en $C_1$-$C_6$) à l'aide d'un hydrure métallique en 2-amino 3-(1H-imidazol-4-yl)-butanol, on transforme ce dérivé de butanol en 2-amino 1-chloro-3-(1H-imidazol-4-yl) butane à l'aide d'un agent de chloration, et on réduit catalytiquement ce dérivé chloré.

6. Procédé selon la revendication 3, caractérisé en ce que l'agent de chloration est le chlorure de thionyle.

### Revendications pour l'Etat contractant suivant : ES

1. Procédé de préparation de l'α,β-diméthylhistamine, sous forme racémique, d'isomère optique ou de mélange diastéréoisomérique, ainsi que ses sels d'addition à des acides pharmaceutiquement acceptables, caractérisé en ce que l'on réduit un 2-amino 3-(1H-imidazol-4-yl)-butyrate d'alkyle (en $C_1$-$C_6$) à l'aide d'un hydrure métallique en 2-amino 3-(1H-imidazol-4-yl)-butanol, on transforme ce dérivé de butanol en 2-amino 1-chloro-3-(1H-imidazol-4-yl) butane à l'aide d'un agent de chloration, et on réduit catalytiquement ce dérivé chloré.

2. Procédé selon la revendication 1, caractérisé en ce que l'agent de chloration est le chlorure de thionyle.

3. Procédé de préparation stéréospécifique de l'isomère(+)-érythro-α,β-diméthylhistamine, ainsi que ses sels d'addition à des acides pharmaceutiquement acceptables, caractérisé en ce que l'on transformeun (1H-imidazol-4-yl)-carboxylate d'alkyle (en $C_1$-$C_6$), dont le reste imidazole est protégé (notamment par un groupe triphénylméthyle), en érythro-α,β-diméthylhistamine et on sépare les énantiomères (+) et (-) de

ce composé au moyen d'un acide optiquement actif.

**Revendications pour l'Etat contractant suivant : GR**

1. L'α,β-diméthylhistamine, sous forme racémique, d'isomère optique ou de mélange diastéréoisomérique, ainsi que ses sels d'addition à des acides pharmaceutiquement acceptables.

2. L'érythro-α,β-diméthylhistamine, ainsi que ses sels d'addition à des acides pharmaceutiquement acceptables.

3. L'isomère(+)-érythro-α,β-diméthylhistamine, ainsi que ses sels d'addition à ces acides pharmaceutiquement acceptables.

4. Procédé de préparation du composé selon la revendication 1, caractérisé en ce que l'on réduit un 2-amino 3-(1H-imidazol-4-yl)-butyrate d'alkyle (en $C_1$-$C_6$) à l'aide d'un hydrure métallique en 2-amino 3-(1H-imidazol-4-yl)-butanol, on transforme ce dérivé de butanol en 2-amino 1-chloro-3-(1H-imidazol-4-yl) butane à l'aide d'un agent de chloration, et on réduit catalytiquement ce dérivé chloré.

5. Procédé selon la revendication 4, caractérisé en ce que l'agent de chloration est le chlorure de thionyle.

**Patentansprüche**

**Patentansprüche für folgende Vertragsstaaten : AT, BE, CH, DE, FR, GB, IT, LI, LU, NL, SE**

1. α,β-Dimethylhistamin, als racemische Form des optischen Isomers oder der Diastereomerenmischung, sowie dessen pharmazeutisch annehmbare Säureadditionssalze.

2. erythro-α,β-Dimethylhistamin, sowie dessen pharmazeutisch annehmbare Säureadditionssalze.

3. (+)-Isomer von erythro-α,β-Dimethylhistamin, sowie dessen pharmazeutisch annehmbare Säureadditionssalze.

4. Pharmazeutische Zusammensetzung, enthaltend eine Verbindung nach einem der Ansprüche 1 bis 3 und ein pharmazeutisch verträgliches Vehikel oder eine pharmazeutisch verträgliche Trägersubstanz.

5. Verfahren zur Herstellung der Verbindung nach Anspruch 1, dadurch gekennzeichnet, daß man ein Alkyl(mit $C_1$-$C_6$)-2-amino-3-(1H-imidazol-4-yl)butyrat mit Hilfe eines Metallhydrids zu 2-Amino-3-(1H-imidazol-4-yl)butanol reduziert, dieses Butanol-Derivat mit Hilfe eines Chlorierungsmittels in 2-Amino-1 -chlor-3-(1H-imidazol-4-yl)butan umwandelt und man dieses chlorierte Derivat katalytisch reduziert.

6. Verfahren nach Anspruch 3, dadurch gekennzeichnet, daβ das Chlorierungsmittel Thionylchlorid ist.

**Patentansprüche für folgenden Vertragsstaat : ES**

1. Verfahren zur Herstellung von α,β-Dimethylhistamin, als racemische Form des optischen Isomers oder der Diastereomerenmischung, sowie dssen pharmazeutisch annehmbarer Säureadditionssalze, dadurch gekennzeichnet, daβ man ein Alkyl(mit $C_1$-$C_6$)-2-amino-3-(1H-imidazol-4-yl)butyrat mit Hilfe eines Metallhydrids zu 2-Amino-3-(1H-imidazol-4-yl)butanol reduziert, dieses Butanol-Derivat mit Hilfe eines Chlorierungsmittels in 2-Amino-1-chlor-3-(1H-imidazol-4-yl)butan umwandelt und man dieses chlorierte Derivat katalytisch reduziert.

2. Verfahren nach Anspruch 1, dadurch gekennzeichnet, daβ das Chlorierungsmittel Thionylchlorid ist.

3. Verfahren zur stereospezifischen Herstellung des (+)-Isomers von erythro-α,β-Dimethylhistamin, sowie dessen pharmazeutisch annehmbarer Säureadditionssalze, dadurch gekennzeichnet, daβ man ein Alkyl(mit $C_1$-$C_6$)-(1H-imidazol-4-yl)carboxylat, dessen Imidazol-Rest geschützt ist (insbesondere durch eine Triphenylmethyl-Gruppe), in erythro-α,β-Dimethylhistamin umwandelt und man die Enantiomeren (+) und (-) dieser Verbindung mittels einer optisch aktiven Säure trennt.

**Patentansprüche für folgenden Vertragsstaat : GR**

1. α,β-Dimethylhistamin, als racemische Form des optischen Isomers oder der Diastereomerenmischung, sowie dessen pharmazeutisch annehmbare Säureadditionssalze.

2. erythro-α,β-Dimethylhistamin, sowie dessen pharmazeutisch annehmbare Säureadditionssalze.

3. (+)-Isomer von erythro-α,β-Dimethylhistamin, sowie dessen pharmazeutisch annehmbare Säureadditionssalze.

4. Verfahren zur Herstellung der Verbindung nach Anspruch 1, dadurch gekennzeichnet, daβ man ein Alkyl(mit $C_1$-$C_6$)-2-amino-3-(1H-imidazol-4-yl)butyrat mit Hilfe eines Metallhydrids zu 2-Amino-3-(1H-imidazol-4-yl)butanol reduziert, dieses Butanol-Derivat mit Hilfe eines Chlorierungsmittels in 2-Amino-1-chlor-3-(1H-imidazol-4-yl)butan umwandelt und man dieses chlorierte Derivat katalytisch reduziert.

5. Verfahren nach Anspruch 4, dadurch gekennzeichnet, daβ das Chlorierungsmittel Thionylchlorid ist.

**Claims**

**Claims for the following Contracting States : AT, BE, CH, DE, FR, GB, IT, LI, LU, NL, SE**

1. α,β-dimethylhistamine, in racemic form or in the form of an optical isomer or a diastereoisomeric mixture, and pharmaceutically acceptable acid addition salts thereof.

2. Erythro-α,β-dimethylhistamine, and pharmaceutically acceptable acid addition salts thereof.

3. The isomer (+)-erythro-α,β-dimethylhistamine, and pharmaceutically acceptable acid addition salts thereof.

4. A pharmaceutical composition containing a compound according to any one of Claims 1 to 3 and a pharmaceutically compatible vehicle or excipient.

5. A process for the preparation of the compound according to Claim 1, characterised in that a $C_{1-6}$-alkyl-2-amino-3-(1H-imidazol-4-yl)butyrate is reduced by means of a metal hydride into 2-amino-3-(1H-imidazol-4-yl)butanol, this butanol derivative is converted into 2-amino-1-chloro-3-(1H-imidazol-4-yl)butane by means of a chlorinating agent, and this chlorinated derivative is reduced catalytically.

6. A process according to Claim 3, characterised in that the chlorinating agent is thionyl chloride.

**Claims for the following Contracting State : ES**

1. A process for the preparation of α,β-dimethylhistamine, in racemic form or in the form of an optical isomer or a diastereoisomeric mixture, and pharmaceutically acceptable acid addition salts thereof, characterised in that a $C_{1-6}$-alkyl-2-amino-3-(1H-imidazol-4-yl)butyrate is reduced by means of a metal hydride into 2-amino-3-(1H-imidazol-4-yl)butanol, this butanol derivative is converted into 2-amino-1-chloro-3-(1H-imidazol-4-yl)butane by means of a chlorinating agent, and this chlorinated derivative is reduced catalytically.

2. A process according to Claim 1, characterised in that the chlorinating agent is thionyl chloride.

3. A process for the stereospecific preparation of the isomer (+)-erythro-α,β-dimethylhistamine, and pharmaceutically acceptable acid addition salts thereof, characterised in that a $C_{1-6}$-alkyl-(1H-imidazol-4-yl)-carboxylate, whose imidazol residue is protected (in particular by a triphenylmethyl group), is converted into erythro-α,β-dimethylhistamine and the (+) and (-) enantiomers of this compound are separated by means of an optically active acid.

**Claims for the following Contracting State : GR**

1. α,β-dimethylhistamine, in racemic form or in the form of an optical isomer or a diastereoisomeric mixture,

and pharmaceutically-acceptable acid addition salts thereof.

2. Erythro-α,β-dimethylhistamine, and pharmaceutically acceptable acid addition salts thereof.

3. The isomer (+)-erythro-α,β-dimethylhistamine, and pharmaceutically acceptable addition salts thereof.

4. A process for the preparation of the compound according to Claim 1, characterised in that a $C_{1-6}$-alkyl-2-amino-3-(1H-imidazol-4-yl)butyrate is reduced by means of a metal hydride into 2-amino-3-(1H-imidazol-4-yl)butanol, this butanol derivative is converted into 2-amino-1-chloro-3-(1H-imidazol-4-yl)butane by means of a chlorinating agent, and this chlorinated derivative is reduced catalytically.

5. A method according to Claim 4, characterised in that the chlorinating agent is thionyl chloride.